(19) Europäisches Patentamt
European Patent Office
Office européen des brevets

(11) EP 1 614 411 B1

(12) EUROPEAN PATENT SPECIFICATION

(45) Date of publication and mention of the grant of the patent:
**05.10.2011 Bulletin 2011/40**

(21) Application number: **03710419.7**

(22) Date of filing: **19.03.2003**

(51) Int Cl.:
*A61Q 1/02* (2006.01) *A61K 8/31* (2006.01)
*A61K 8/37* (2006.01) *A61K 8/39* (2006.01)

(86) International application number:
**PCT/JP2003/003295**

(87) International publication number:
**WO 2004/082645 (30.09.2004 Gazette 2004/40)**

(54) **OILY MAKEUP COSMETICS**

ÖLIGE MAKEUP-KOSMETIKA

COMPOSITIONS DE MAQUILLAGE A BASE D'HUILE

(84) Designated Contracting States:
**FR**

(43) Date of publication of application:
**11.01.2006 Bulletin 2006/02**

(73) Proprietors:
- **Kanebo Cosmetics Inc.**
  **Tokyo 105-8085 (JP)**
- **The Nisshin Oillio Group, Ltd.**
  **Chuo-ku**
  **Tokyo 104-8285 (JP)**

(72) Inventors:
- **EGAWA, Yuichiro**
  **Odawara-shi, Kanagawa 250-0055 (JP)**
- **HISHIKAWA, Noboru**
  **Hanamigawa-ku, Chiba-shi, Chiba 262-0032 (JP)**

(74) Representative: **Corizzi, Valérie et al**
**Cabinet ORES,**
**36, rue de Saint Pétersbourg**
**75008 Paris (FR)**

(56) References cited:
**WO-A-02/069889** **WO-A1-03/015741**
**JP-A- 2001 226 223** **JP-A- 2002 316 910**
**JP-A- 2002 370 930** **JP-A- 2003 104 843**
**US-A- 5 436 006** **US-A- 5 919 398**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

Technical field

The present invention relates to oily makeup cosmetics, and is to provide oily makeup cosmetics which direct smooth feeling or excellent gloss at the time of coating, and are excellent in stability with a lapse of time.

Background art

Heretofore, it has widely been used to use a hydrocarbon having a branched structure such as polybutene, etc. to formulate oily makeup cosmetics which are required to have glowing or gloss at finishing. For the purpose of solidifying or increasing the viscosity of the oily makeup cosmetics containing the hydrocarbon, it has been also well known to use solid wax or dextrin palmitate as a gelling agent. However, when the solid wax, required to provide glowing or gloss, is to be formulated into oily makeup cosmetics, if the formulation amount thereof is low, syneresis (sweating) tends to be caused, and by increasing the formulation amount thereof, the problem is that glowing or gloss at finishing is impaired. Also, dextrin palmitate is an oily gelling agent having an excellent gelling function to a variety of oily basic agents, but it does not effectively perform its gelling function onto polybutene, etc., and as a result, the resulting oily makeup cosmetics difficultly have a desired hardness or viscosity, and a decrease in hardness or viscosity tends to increase with time, and in the worst case, the problem is that the cosmetics separate in the composition. These problems can be solved to some extent by increasing the formulation amount of dextrin palmitate, but the feeling becomes worse, and glowing or gloss is impaired as in the case where solid wax is formulated.

On the other hand, an oligo ester obtained from (a) an aliphatic acid or a hydroxy aliphatic acid each having 8 to 30 carbon atoms (each straight or branched, saturated or unsaturated), (b) a straight or branched dibasic acid having 12 to 36 carbon atoms, (c) glycerol or a glycerol condensate is a conventionally known substance (Japanese Patent Publication No. Shou.61-7402, Japanese Unexamined Patent Publication No. Hei.7-126604), and it has also been reported that it is effective as an emulsifying aid for emulsified preparations (Japanese Unexamined Patent Publication No. 2000-219617). However, in oily makeup cosmetics comprising a hydrocarbon having a branched structure as an essential component, it has not been known that the conventional problems can be solved by formulating the same.

In such an actual circumstance, the present inventors have earnestly studied to solve the above-mentioned problems, and as a result, they have found that oily makeup cosmetics according to claim 1 can be obtained, having an excellent feeling in touch or stability with a lapse of time without impairing excellent gloss-providing effects possessed by the hydrocarbon having a branched structure, whereby the present invention has been accomplished.

Summary of the invention

That is, the present invention relates to oily makeup cosmetics according to claim 1.

Best mode for carrying out the invention

The oligo ester is a compound represented by the following formula, in which (a) is behenic acid, (b) is eicosanoic diacid, (c) is glycerol or a glycerol condensate. They are commercially available as "NOMCORT HK-G", "NOMCORT HK-P" from Nisshin OilliO Co., Ltd. and can be obtained easily. A formulation amount of the above-mentioned oligo ester in the present invention is suitably 0.5 to 10% by weight based on 100% by weight of the resulting oily makeup cosmetics. It is more preferably 1 to 8% by weight, and particularly preferably 2 to 5% by weight. If it is less than 0.5% by weight, effects of formulating a gelling agent are difficultly shown, while if it exceeds 10% by weight, feeling is markedly worsened and thus it is not preferred. Incidentally, the oily cosmetics in the present invention mean those which do not substantially contain water.

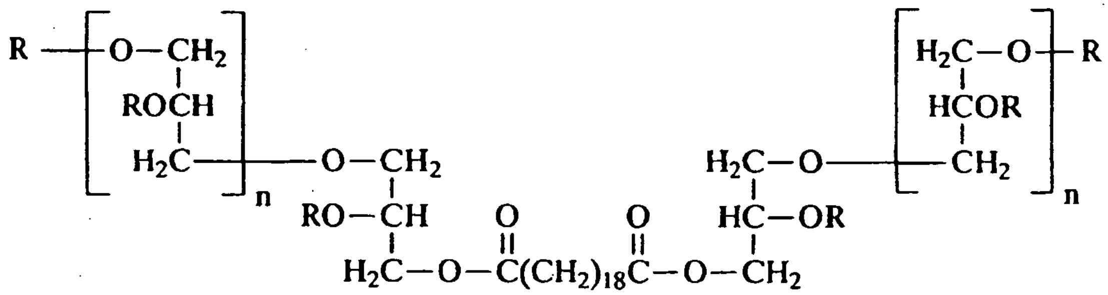

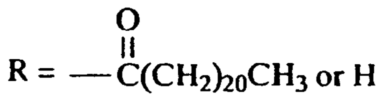

$n = 0 \sim 8$

The hydrocarbon having a branched structure to be used in the present invention is polybutene or heavy liquid isoparaffin (hydrogenated polybutene). Also, the average molecular weight is preferably in the range of 500 to 2700. If the average molecular weight is less than 500, there is a case where it is insufficient to provide glowing or gloss to oily makeup cosmetics at the time of coating, on the other hand, if it exceeds 2700, it is not preferred since there are cases where it becomes difficult to obtain oily makeup cosmetics which are preferred in the points of feeling such as extending property, stickiness, etc. Moreover, when handling is considered, at the time of preparing the oily makeup cosmetics, the range of 800 to 1500 is more preferred. A formulation amount of the hydrocarbon having a branched structure in the present invention is suitably 5 to 80% by weight based on 100% by weight of the resulting oily makeup cosmetics. It is more preferably 15 to 50% by weight, and particularly preferably 20 to 40% by weight. If it is less than 5% by weight, there is a case where it is insufficient to provide glowing or gloss to oily makeup cosmetics at the time of coating, while if it exceeds 80% by weight, extending property, stickiness, etc. are markedly worsened.

In the oily makeup cosmetics of the present invention, other than the above-mentioned essential components, components of various kinds of powders, oily agents, UV ray inhibitors, fluorine compounds, resins, thickening agents, antiseptic agents, flavors, humectants, salts, solvents, antioxidants, chelating agents, insect inhibitors, etc., which are usually formulated in cosmetics may be used within the range which does not impair the effects of the present invention. Examples of the pigments to be used in the present invention are not limited in the shape (spherical, rod shaped, needle shaped, plate shaped, amorphous shaped, scaly shaped, spindle shaped, etc.) or particle size (fumed state, fine particulate, pigment class, etc.), particle structure (porous, non-porous, etc.) so long as it is used in a conventional cosmetics and any of them may be used, and there may be mentioned, for example, inorganic powder, organic powder, surfactant metal salt powder, colored pigment, pearl pigment, metal powder pigment, natural dye, etc., more specifically, as the inorganic powders, there may be mentioned pigment class titanium oxide, zirconium oxide, pigment class zinc oxide, cerium oxide, magnesium oxide, barium sulfate, calcium sulfate, magnesium sulfate, calcium carbonate, magnesium carbonate, talc, mica, kaolin, sericite, muscovite, synthetic mica, phlogopite, lepidolite, biotite, silicic acid, anhydrous silicic acid, aluminum silicate, magnesium silicate, aluminum magnesium silicate, calcium silicate, barium silicate, strontium silicate, tungstic acid metal salt, hydroxyapatite, vermiculite, Higilite(™), bentonite, montmorillonite, hectorite, zeolite, ceramics powder, calcium secondary phosphate, alumina, aluminum hydroxide, boron nitride, silica, fine particulate titanium oxide, fine particulate zinc oxide, fine particulate cerium oxide, etc.; as the organic powders, there may be mentioned polyamide powder, polyester powder, polyethylene powder, polypropylene powder, polystyrene powder, polyurethane powder, benzoguanamine powder, polymethybenzoguanamine powder, polytetrafluoroethylene powder, Polymethyl methacrylate powder, cellulose, silk powder, Nylon powder, 12 Nylon, 6 Nylon, silicone powder, silicone rubber powder, silicone elastomer spherical powder, styrene·acrylic acid copolymer, divinylbenzene·styrene copolymer, vinyl resin, urea resin, phenol resin, fluorine resin, silicone resin, acrylic resin, melamine resin, epoxy resin, polycarbonate resin, microcrystalline fiber powder, starch powder, lauroyl lysine, etc.; as the surfactant metal salt powders (metallic soap), there may be mentioned zinc stearate, aluminum stearate, calcium stearate, magnesium stearate, zinc myristate, magnesium myristate, cetyl zinc phosphate, cetyl calcium phosphate, cetyl zinc sodium phosphate, etc.; as the colored pigments, there may be mentioned inorganic red pigments such as iron oxide, iron hydroxide and iron titanate, inorganic

brown series pigments such as γ-iron oxide, etc., inorganic yellow series pigments such as yellow iron oxide, ocher, etc., inorganic black pigments such as black iron oxide, carbon black, etc., inorganic purple pigments such as manganese violet, cobalt violet, etc., inorganic green pigments such as chromium hydroxide, chromium oxide, cobalt oxide, cobalt titanate, etc., inorganic blue series pigments such as Prussian blue, ultramarine blue, etc., lake of tar series dyes, lake of natural dyes, synthetic resin powders in which these powders are complexed, etc.; as the pearl pigments, there may be mentioned titanium oxide-coated mica, bismuth oxychloride, titanium oxide-coated bismuth oxychloride, titanium oxide-coated talc, fish scale flake, titanium oxide-coated colored mica, etc.; as the tar dyes, there may be mentioned Red No. 104, Red No. 106, Red No. 201, Red No. 202, Red No. 218, Red No. 223, Red No. 226, Yellow No. 4, Yellow No. 5, Yellow No. 401, Blue No. 1, Blue No. 404, Orange No. 201, etc.; as the natural dyes, there may be mentioned powders selected from carminic acid, laccaic acid, carthamine, brazilin, crocin, etc., and as these powders, those in which the powders are complexed or treated with a general oily agents, silicone oils, fluorine compounds, surfactants, etc., may be used within the range which does not prevent the effects of the present invention. For example, they may be provisionally surface treated by a fluorine compound-treatment, silicone resin-treatment, pendant treatment, silane coupling agent-treatment, titan coupling agent-treatment, oily agent-treatment, polyacrylic acid-treatment, metallic soap-treatment, amino acid-treatment, inorganic compound-treatment, plasma treatment, mechanochemical treatment, etc., and depending on necessity, one kind or two or more kinds of the surface treatments may be used in combination.

Examples of the oily agents may include, for example, avocado oil, linseed oil, almond oil, insect (IBOTA) wax, perilla oil, olive oil, cacao butter, kapok wax, Torreya nucifera (kaya) oil, carnauba wax, cod liver oil, candelilla wax, beef tallow, beef foot fat, beef bone fat, hydrogenated beef tallow, apricot kernel oil, spermaceti, hydrogenated oil, wheat germ oil, sesame oil, rice germ oil, rice bran oil, corn wax, sasanqua oil, safflower oil, shea butter, Chinese tung oil, cinnamon oil, jojoba wax, shellac wax, turtle oil, soy bean oil, tea seed oil, camellia oil, evening primrose oil, corn oil, lard, rape seed oil, Japanese tung oil, rice bran wax, germ oil, horse oil, persic oil, palm oil, palm kernel oil, castor oil, hydrogenated castor oil, castor oil fatty acid methyl ester, sunflower oil, grape seed oil, bayberry wax, jojoba oil, macadamia nut oil, beeswax, mink oil, cottonseed oil, cotton wax, Japan wax, Japan kernel oil, montan wax, coconut oil, hydrogenated coconut oil, coconut fatty acid triglyceride, mutton-tallow, peanut oil, lanolin, liquid lanolin, reduced lanolin, lanolin alcohol, hard lanolin, lanolin acetate, lanolin fatty acid isopropyl ester, hexyl laurate, polyoxyethylene (hereinafter abbreviated to as POE) lanolin alcohol ether, POE lanolin alcohol acetate, lanolin fatty acid polyethylene glycol, POE hydrogenated lanolin alcohol ether, egg yolk oil, etc.; as the hydrocarbon oils, there may be mentioned ozokerite, ceresin, liquid paraffin, polyethylene wax, polyglyceryl monoisostearate, (adipic acid-2-ethylhexanoic acid-stearic acid) glyceryl oligo ester, (2-hexyldecanoic acid·sebacic acid)-diglyceryl oligo ester, tri(caprylic·capric·myristic· stearic acid)glyceride, ethylene-propylene copolymer, microcrystalline wax, Vaseline, etc.; as the higher fatty acids, there may be mentioned lauric acid, myristic acid, palmitic acid, stearic acid, behenic acid, undecylenic acid, oleic acid, linoleic acid, linolenic acid, arachidonic acid, eicosapentaenoic acid (EPA), docosahexaenoic acid (DHA), isostearic acid, 12-hydroxystearic acid, etc.; as the higher alcohols, there may be mentioned lauryl alcohol, myristyl alcohol, palmityl alcohol, stearyl alcohol, behenyl alcohol, hexadecyl alcohol, oleyl alcohol, isostearyl alcohol, hexyldodecanol, octyldodecanol, ceto stearyl alcohol, 2-decyl tetradecanol, cholesterol, phytosterol, POEcholesterolether, monostearyl glycerin ether (batyl alcohol), monooleyl glyceryl ether (selachyl alcohol), etc.; as the ester oils, there may be mentioned diisobutyl adipate, 2-hexyldecyl adipate, di-2-heptylundecyl adipate, isononyl isononanate, isotridecyl isononanate, N-alkylglycol monoisostearate, isocetyl isostearate, trimethylolpropane triisostearate, neopentyl glycol di-2-ethylhexanoate, cetyl 2-ethylhexanoate, trimethylolpropane tri-2-ethylhexanoate, polyglyceryl diisostearate, polyglyceryl triisostearate, polyglyceryl tetraisostearate, pentaerythritol tetra-2-ethylhexanoate, cetyl octanoate, octyldodecyl rubber ester, oleyl oleate, octyldodecyl oleate, decyl oleate, phytosteryl oleate, neopentyl glycol dicaprate, 2-ethylhexyl succinate, isocetyl stearate, butyl stearate, diisopropyl sebacate, di-2-ethylhexyl sebacate, cetyl lactate, myristyl lactate, isopropyl palmitate, 2-ethylhexyl palmitate, 2-hexyldecyl palmitate, 2-heptylundecyl palmitate, octyl palmitate, cholesteryl 12-hydroxystearate, dipentaerythritol fatty acid ester, isopropyl myristate, octyldodecyl myristate, isostearyl myristate, 2-hexyldecyl myristate, myristyl myristate, hexyldecyl dimethyloctanoate, ethyl laurate, hexyl laurate, di(cholesteryl·octyldodecyl) N-lauroyl-L-glutamate, di(cholesteryl·behenyl·octyldodecyl) N-lauroyl-L-glutamate, di(phytosteryl·octyldodecyl) N-lauroyl-L-glutamate, di(phytosteryl·behenyl·octyldodecyl) N-lauroyl-L-glutamate, diisostearyl malate, glyceryl triisooctanoate, tri(capryl·capric acid) glycerin, glyceryl triisostearate, glyceryl triisopalmitate, glyceryl monostearate, glyceryl di-2-heptylundecanoate, glyceryl trimyristate, diglyceryl myristate isostearate, etc.

Also, examples of the other form of oily agents, there may be mentioned, for example, silicone compounds such as dimethylpolysiloxane, methyl hydrogen polysiloxane, methylphenyl polysiloxane, alkyl-modified organopolysiloxane, terminal-modified organopolysiloxane, fluorine-modified organopolysiloxane, amino-modified organopolysiloxane, polyether-modified silicone, perfluoroalkyl·polyoxyalkylene co-modified organopolysiloxane, acryl-modified silicone, glyceryl-modified silicone, polyglyceryl-modified silicone, sugar-modified silicone, silicone gel, silicone RTV rubber, etc., fluorine compounds such as perfluoropolyether, fluorinated pitch, fluorocarbon, fluoro alcohol, etc.

As an ultraviolet ray-inhibiting component, there may be mentioned inorganic series and organic series ultraviolet ray inhibiting agents. Examples of the inorganic series ones, there may be mentioned, for example, metal oxides

such as titanium dioxide, titanium low-level oxide, zinc oxide, cerium oxide, etc., metal hydroxides such as iron hydroxide, etc., metal flakes such as plate iron oxide, aluminum flake, etc., ceramics such as silicon carbide, etc. Of these, it is particularly preferred that the material is at least one selected from fine particulate metal oxide and fine particulate metal hydroxide each having an average particle size of 5 to 100 nm. These powders are preferably subjected to surface treatment by the conventionally known surface treatment, for example, fluorine compound treatment (perfluoroalkylphosphoric acid ester treatment, perfluoroalkylsilane treatment, perfluoropolyether treatment, fluorosilicone treatment, fluorinated silicone resin treatment is preferred), silicone treatment (methylhydrogenpolysiloxane treatment, dimethylpolysiloxane treatment, vapor phase method tetramethyltetrahydrogen-cyclotetrasiloxane treatment is preferred), silicone resin treatment (trimethylsiloxysilicic acid treatment is preferred), pendant treatment (a method of adding alkyl chain, etc., after vapor phase method-silicone treatment), silane coupling agent treatment, titanium coupling agent treatment, silane treatment (alkylsilane or alkylsilazane treatment is preferred), oily agent treatment, N-allylated lysine treatment, polyacrylic acid treatment, metal soap treatment (stearic acid or myristic acid salt is preferred), acryl resin treatment, metal oxide treatment, etc., more preferably, these treatments are carried out by combining a plural kind thereof. For example, there may be mentioned a method in which fine particulate titanium oxide surface is coated with silicon oxide or a metal oxide such as alumina, etc., and then subjected to surface treatment with alkyl silane and the like. An amount of the surface treatment is preferably within the range of 0.1 to 50% by weight in a total amount of the surface treatment based on the weight of the powder.

Also, examples of the organic series UV ray inhibitors, there may be mentioned, for example, glyceryl mono-octanoate di-p-methoxy cinnamate, 2-ethylhexyl p-methoxy cinnamate (another name; octyl p-methoxy cinnamate), 2-hydroxy-4-methoxybenzophenone, 2-hydroxy-4-methoxybenzophenone-5-sulfuric acid, 2,2'-dihydroxy-4-methoxybenzophenone, p-methoxyhydrocinnamate diethanolamine salt, p-aminobenzoic acid (hereinafter abbreviated to as PABA), ethyldihydroxypropyl PABA, glyceryl PABA, homomenthyl salicylate, methyl-O-aminobenzoate, 2-ethylhexyl-2-cyano-3,3-diphenylacrylate, octyl dimethyl PABA, octyl salicylate, 2-phenyl-benzimidazol-5-sulfate, triethanolamine salicylate, 3-(4-methylbenzylidene)camphor, 2,4-dihydroxybenzophenone, 2,2',4,4'-tetrahydroxybenzophenone, 2,2'-dihydroxy-4,4'-dimethoxybenzophenone, 2-hydroxy-4-N-octoxy-benzophenone, 4-isopropyl dibenzoylmethane, butylmethoxydibenzoylmethane, octyltriazone, 4-(3,4-dimethoxyphenyl-methylene)-2,5-dioxo-1-imidazolidine 2-ethylhexyl propionate, and polymer derivatives and silane derivatives of the above-mentioned compounds, etc. Also, it is possible to use those in which the organic series UV ray inhibitors are encapsulated into polymer powder. The polymer powder may be hollow or not may be hollow, and as an average primary particle size may be within the range of 0.1 to 50 μm, and the particle size distribution may be broad or may be shape. As a kind of the polymer, there may be mentioned an acrylic resin, a methacrylic resin, a styrene resin, a polyurethane resin, polyethylene, polypropylene, polyethylene terephthalate, a silicone resin, Nylon, an acrylamide resin, etc. Preferred are powder in which organic series UV ray inhibitors are incorporated in these polymer powders in an amount in the range of 0.1 to 30% by weight based on the weight of the powder, particularly preferably 4-tert-butyl-4'-methoxydibenzoylmethane which is a UVA absorbent is formulated. Among the above-mentioned UV ray inhibitor components, it is preferred to use at least one selected from the group consisting of fine particulate titanium oxide, fine particulate zinc oxide, 2-ethylhexyl paramethoxycinnamate, butylmethoxydibenzoylmethane, oxybenzone and benzophenone series UV ray absorbent is generally used, commercially available, and has high UV ray inhibiting effects. In particular, it is preferred to use the inorganic series and organic series ones in combination. Also, it is suitable to use that corresponding to UV-A and that corresponding to UV-B in combination.

Examples of the thickening agents may include vegetable series polymers such as acacia, tragacanth, arabinogalactan, locust bean gum (carob gum), guar gum, karaya gum, carrageenan, pectin, agar, quince seed (quince), starch (rice, corn, potato, wheat), algaecolloid, gum tragacanth, locust bean gum, etc., microorganism series polymers such as xanthan gum, dextran, succinoglucan, pullulan, etc., animal series polymers such as collagen, casein, albumin, etc., starch series polymers such as carboxymethyl starch, methylhydroxypropyl starch, etc., cellulose series polymers such as methyl cellulose, ethyl cellulose, methylhydroxypropyl cellulose, carboxymethyl cellulose, hydroxymethyl cellulose, hydroxypropyl cellulose, nitro cellulose, sodium sulfate cellulose, sodium carboxymethyl cellulose, crystalline cellulose, cellulose powder, etc., alginic acid series polymers such as sodium alginate, alginic acid propylene glycol ester, etc., vinyl series polymers such as polyvinylmethyl ether, polyvinylpyrrolidone, carboxyvinyl polymer, etc., polyoxyethylene series polymers such as polyethylene glycol, etc., acrylic series polymers such as polyoxyethylene polyoxypropylene copolymer series polymer, sodium polyacrylate, polyethyl acrylate, polyacrylic acid amide, etc., polyethyleneimine, cationic polymer, inorganic series thickening agents such as bentonite, aluminum magnesium silicate, laponite, smectite, saponite, hectorite, anhydrous silicic acid, etc. Also, as the other thickening agents, there is an oil-soluble gelling agent, and may be mentioned, for example, metal soaps such as aluminum stearate, magnesium stearate, zinc myristate, etc., amino acid derivatives such as N-lauroyl-L-glutamic acid, $\alpha,\gamma$-di-n-butylamine, etc., dextrin fatty acid esters such as dextrin palmitic acid ester, dextrin stearic acid ester, dextrin 2-ethylhexanoic acid palmitic acid ester, etc., sucrose fatty acid esters such as sucrose palmitic acid ester, sucrose stearic acid ester, etc., benzylidene derivatives of sorbitol such as mono-benzylidene sorbitol, dibenzylidene sorbitol, etc., organic modified clay minerals such as dimethylbenzyldodecyl ammonium montmorillonite clay, dimethyldioctadecyl ammonium montmorillonite, octadecyldimethylbenzyl ammonium montmorillonite, etc.

As the physiologically active components to be used in the present invention, substances which provide any physiological activity to skin when it is coated onto skin may be mentioned. For example, there may be mentioned anti-inflammatory agents, anti-aging agents, astringents, humectants, blood circulation promoters, antibacterial agents, bactericides, dryers, cooling agents, warming agents, vitamins, amino acids, wound healing promoters, torpents, analgesics, cell activating agents, enzyme components, etc. Of these, natural series vegetable extracted components, seaweed extracted components, crude drug components are particularly preferred. In the present invention, one kind or two or more kinds of these physiologically active components is/are preferably formulated. For example, there may be mentioned angelica extract, locust (acacia) extract, silvervine extract, avocado extract, hydrangea extract, althea extract, arnica extract, aloe extract, apricot extract, apricot kernel extract, ginkgo extract, turmeric extract, woolong tea extract, rose fruit extract, Echinacea leaf extract, Scutellaria root extract, phellodendron bark extract, barley extract, horse chestnut extract, leek extract, hollyhock extract, silver-mound artemisia extract, sea-bindweed extract, green tea extract, marguerite extract, cinnamon extract, tear grass extract, Hypericum extract, white nettle extract, water-cress extract, orange extract, saffron extract, loquat extract, sea salt, hydrolyzed elastin, hydrolyzed wheat powder, hydrolyzed silk, cassis extract, sun spurge extract, fennel extract, golden bells extract, gentian extract, chamomile extract, carrot extract, Artemisia capillaris extract, hibiscus extract, kiwi extract, geranium herb extract, ganoderma extract, cinchona extract, cucumber extract, guanosine, gardenia extract, hotarukazura (*Lithospermum zollingeri*) extract, lemon balm extract, olive extract, Oenothera erythrosepara extract, *Sasa* Albomarginata extract, sophora extract, walnut extract, grape fruit extract, plantain extract, Japanese knotweed extract, Sambucus nigra extract, clematis extract, Rhei rhizoma extract, great duckweed extract, chickweed extract, clove extract, nettle extract, thyme extract, chlorella extract, mulberry bark extract, gentian extract, black tea extract, yeast extract, burdock root extract, fermented rice bran extract, rice germ oil, comfrey extract, collagen, bilberry extract, Asiasarum root extract, Bupleuri radix extract, umbilical cord extract, salvia extract, Saponaria extract, bamboo grass extract, Crataegus Fruit extract, Zanthoxylum fruit extract, shiitake extract, Rehmannia root extract, Lithospermum root extract, perilla herb extract, linden extract, Filipendula extract, peony root extract, Calamus rhizome extract, birch extract, horsetail extract, ivy extract, Crataegus extract, Sambucus extract, yarrow extract, peppermint extract, sage extract, mallow extract, Cnidium rhizome extract, Swertia herb extract, soy extract, jujube extract, thyme extract, cogon grass extract, citrus unshiu peel extract, Japanes angelica root extract, calendula extract, perch seed extract, bitter orange peel extract, Houttuynia extract, tomato extract, Natto extract, ginseng extract, garlic extract, wild rose extract, hibiscus extract, Ophiopogon extract, lotus extract, parsley extract, honey, pellitory extract, Isodonis extract, bisabolol, coltsfoot extract, butterbur scape extract, hoelen extract, Butcher broom extract, grape extract, propolis, sponge gourd extract, safflower extract, peppermint extract, linden extract, Paeonia extract, hop extract, pine extract, skunk cabbage extract, Mukurossi peel extract, peach extract, cornflower extract, eucalyptus extract, saxifrage extract, *yuzu* (*Citrus junos*) extract, coix extract, mugwort extract, lavender extract, lettuce extract, lemon extract, Chinese milk vetch extract, rose extract, romanchamomile extract, royal jelly extract, etc.

Also, there may be mentioned biopolymers such as mucopolysaccharide, sodium hyaluronate, chondroitin sodium sulfate, collagen, elastin, chitin, chitosan, hydrolyzed egg shell membrane, etc., amino acids such as glycine, valine, leucine, isoleucine, serine, threonine, phenylalanine, alginine, lysine, aspartic acid, glutamic acid, cystine, cysteine, methionine, triptophane, etc., hormones such as estradiol, ethenyl estradiol, etc., humectants such as amino acid, sodium lactate, urea, sodium pyrrolidone carboxylate, betaine, whey, etc., oily components such as sphingolipids, ceramide, cholesterol, cholesterol derivatives, phospholipids, etc., anti-inflammatory agents such as ε-aminocaprioc acid, glycyrrhizic acid, β-glycyrehtinic acid, lysozyme chloride, guaiazulene, hydrocortisone, allantoin, tranexamic acid, azulene, etc., active components such as Vitamins A, B2, B6, C, D, K, vitamin C glycosides, calcium pantothenate, biotin, nicotinic acid amide, allantoin, diisopropylamine dichloroacetate, 4-aminomethylcyclohexane carboxylic acid, etc., cell activators such as α-hydroxy acid, β-hydroxy acid, etc., blood circulation promoters such as γ-oryzanol, etc., wound healing agents such as retinol, retinol derivatives, etc., refrigerants such as cepharanthine, glycyrrhiza extract, capsicum tincture, hinokitiol, iodized garlic extract, pyridoxine hydrochloride, nicotinic acid, nicotinic acid derivatives, calcium pantothenate, D-pantothenyl alcohol, acetyl pantothenyl ethyl ether, biotin, allantoin, isopropylmethylphenol, estradiol, ethenyl estradiol, capronium chloride, benzalkonium chloride, diphenhydramine hydrochloride, takanal, camphor, salicylic acid, nonylic vanillyl amide, nonanoic acid vanillylamide, piroctone olamine, glyceryl pentadecanoate, 1-menthol, etc.

As oily makeup cosmetics of the present invention, there may be mentioned lipstick, lip gloss, lip cream, foundation, concealer, eye shadow, eye gloss, eyeliner, eyebrow, etc. Moreover, a shape of the products is not specifically limited and may be applied to a liquid state, a stick state, a solid state, a paste state, etc.

EXAMPLES

In the following, the present invention is explained in more detail by referring to Examples and Comparative examples. Incidentally, as the (behenic acid/eicosanoic diacid)glyceryl in the following prescription, "NOMUCORT HK-G" available from Nisshin OilliO was used.

Evaluation methods with regard to various characteristics of the respective oily makeup cosmetics in Example and Comparative example are shown below.

[Evaluation of skin availability]

Each 20 member of special panelists were prepared (provided that panelists are sometimes overlapped with the items to be tested) with regard to the respective evaluation items, and from the number of the panelists judged as excellent in the respective evaluation items, evaluation was carried out according to the classifications mentioned below.

| Number of persons answered to "good" among 20 persons | Evaluation |
|---|---|
| 15 persons or more | ◎ |
| 10 to 14 persons | ○ |
| 5 to 9 persons | Δ |
| 0 to 4 persons | X |

[Hardness]

Oily makeup cosmetics were degassed and then filled in an ointment bottle with a volume of 10 mL at 100˚C, solidified by allowing to stand for cooling, allowed to stand in a thermostat at 30˚C for 6 hours or longer, and the peak value measured by RHEOMETER (RHEO TEX manufactured by Sun Scientific Co., Ltd., plunger diameter: 10 mm, rest elevating rate: 1 mm/sec) was made a hardness.

[Stability]

Oily makeup cosmetics were charged in respective thermostat chambers at 5˚C, 30˚C and 45˚C, for 3 months and observed whether any change in quality occurred or not, and judged when absolutely no problem occurred as O, that which can observe any sigh causing abnormality as Δ, and that caused abnormality as ×.

Example 1, Comparative examples 1 to 2 (Solid oily lipstick of flow-in type)

In the composition (numerals are % by weight) shown below:

| | |
|---|---|
| Polybutene | 25.0 |
| Diisostearyl malate | 25.0 |
| 2-Ethylhexyl hydroxystearate | remainder |
| Liquid paraffin | 5.0 |
| Isotridecyl isononanate | 3.0 |
| Red No. 202 | 0.4 |
| Titanium oxide | 1.0 |
| Red ocher-coated titanium-mica | 5.0 |

investigation was carried out to determine which amounts of (behenic acid/eicosanoic diacid)glyceryl, ceresin and dextrin palmitate should be added to the solid oily lipstick as gelling agents so that the degree of adhesion of the solid oily lipstick to a lip brush or fingers becomes optimum (around 200 g according to the above-mentioned hardness measurement method), and according to the results thereof, solid oily lipsticks shown in Table 1 were prepared according to the conventional manner, then, they were each poured into a resin-made compact vessel, and various kinds of evaluations were carried out. The evaluation results are also shown in Table 1.

Table 1

| | Example | Comparative example | |
|---|---|---|---|
| | 1 | 1 | 2 |
| (Behenic acid/eicosanoic diacid)-glyceryl | 4.0 | | |
| Cerecin | | 8.0 | |

(continued)

| | Example | Comparative example | |
|---|---|---|---|
| | 1 | 1 | 2 |
| Dextrin palmitate | | | 12.0 |
| Polybutene | 25.0 | 25.0 | 25.0 |
| Diisostearyl malate | 25.0 | 25.0 | 25.0 |
| 2-Ethylhexyl hydroxystearate | Remainder | Remainder | Remainder |
| Liquid paraffin | 5.0 | 5.0 | 5.0 |
| Isotridecyl isononanate | 3.0 | 3.0 | 3.0 |
| Red No. 202 | 0.4 | 0.4 | 0.4 |
| Titanium oxide | 1.0 | 1.0 | 1.0 |
| Red ocher-coated titanium-mica | 5.0 | 5.0 | 5.0 |
| Gloss at the time of coating | ◎ | ○ | Δ |
| Feeling | ◎ | Δ | × |
| Storage stability | ○ | × (※) | ○ |
| Hardness | 210 | 200 | 200 |
| ※ : sweating at 30˚C and 45˚C | | | |

As can be clearly seen from the results in Table 1, the lipstick of the present invention was excellent in gloss and feeling at the time of coating and storage stability.

Examples 2 to 3, Comparative examples 3 to 5 (Liquid lip gloss)

According to the prescription shown in Table 2, liquid lip glosses were prepared according to the conventional manner, and then filled in a bottle vessel with a lip brush, and various kinds of evaluations were carried out. The evaluation results are also shown in Table 2.

Table 2

| | Example | | Comparative example | | |
|---|---|---|---|---|---|
| | 2 | 3 | 2 | 3 | 4 |
| Glyceryl(behenic acid/ eicosanoic diacid) | 0.5 | 2.0 | | | |
| Dextrin palmitate | | | 5.0 | 5.0 | |
| Hydrogenated polybutene | 80.0 | 40.0 | 80.0 | 40.0 | 90.0 |
| Diisostearyl malate | | 20.0 | | 20.0 | |
| 2-Ethylhexyl hydroxystearate | Remainder | Remainder | Remainder | Remainder | Remainder |
| Methyl phenyl polysiloxane | | 3.0 | | 3.0 | |
| $\alpha$-Olefin oligomer | 5.0 | 5.0 | 5.0 | 5.0 | |
| Lauroyl lysine-treated Red No. 201 | 0.1 | 0.1 | 0.1 | 0.1 | 0.1 |
| Lauroyl lysine-treated Yellow No. 4 | 0.1 | 0.1 | 0.1 | 0.1 | 0.1 |
| Lauroyl lysine-treated titanium-mica | 2.0 | 2.0 | 2.0 | 2.0 | 2.0 |
| Gloss at the time of coating | ◎ | ◎ | ○ | Δ | ◎ |
| Feeling | ○ | ◎ | Δ | ○ | × |

(continued)

| | Example | | Comparative example | | |
|---|---|---|---|---|---|
| | 2 | 3 | 2 | 3 | 4 |
| Storage stability | ○ | ○ | × ( 1) | Δ ( 2) | × |
| ※ 1: Separated and pigment precipitated at 45˚C<br>※ 2: Seems to be separated at45˚C | | | | | |

As can be clearly seen from the results in Table 2, lip glosses of the present invention were excellent in gloss and feeling at the time of coating and storage stability.

Example 4 (Solid eye gloss of flow-in type)

| | |
|---|---|
| Glyceryl (behenic acid/eicosanoic diacid) | 10.0 |
| Microcrystalline wax | 2.0 |
| Dextrin palmitate | 2.0 |
| Polybutene | 5.0 |
| Glyceryl triocanoate | remainder |
| Liquid paraffin | 20.0 |
| Jojoba oil | 10.0 |
| Propylene glycol dicaprylate | 10.0 |
| Isononyl isononanate | 5.0 |
| Methylpolysiloxane | 10.0 |
| Silica beads | 10.0 |
| Polyalkyl acrylate | 3.0 |
| Red No. 226 | 0.1 |
| (PET/Al/epoxy resin) laminated powder | 5.0 |

Eye gloss of Example 4 was excellent in gloss and feeling at the time of coating and storage stability.

Example 5 (lipstick)

| | |
|---|---|
| Titanium-mica | 3.0 |
| Aluminum lake of Red No. 104(1) | 0.4 |
| Aluminum lake of Blue No. 1 | 0.5 |
| Orange No. 201 | 0.2 |
| Ceresin | 8.0 |
| Microcrystalline wax | 5.0 |
| Paraffin | 4.0 |
| Hydrogenated polybutene | 25.0 |
| Cetyl isooctanoate | 2.0 |
| Glyceryl (behenate/eicosanoic diacid) | 5.0 |
| Vegetable squalane | 0.5 |
| Natural vitamin E | 0.5 |
| Octyl oxystearate | 15.0 |
| octyldodecanol | 5.0 |
| Tri(caprylic·capric·myristic·stearic acid)glyceride | 5.0 |
| Tri(caprylic·capric acid)glycerin | 10.0 |

(continued)

| | |
|---|---|
| Octyldodecyl ricinoleate | 10.9 |
| Example 6 (Foundation) | |
| Candelilla wax | 2.0 |
| Carnauba wax | 1.0 |
| Glyceryl (behenate/eicosanoic diacid) | 0.5 |
| Polyethyleneglycol monostearate (45E.O.) | 0.6 |
| methylpolysiloxane (20cs) | 6.0 |
| Diglycerin oleate | 1.5 |
| Liquid isoparaffin (available from Nippon Oil & Fats Co., Ltd., Parleam 6) | 15.0 |
| Polybutene | 5.0 |
| 2-Ethylhexyl p-dimethylbenzoate | 1.0 |
| Isopropyl isostearate | remainder |
| Anhydrous silicic acid | 0.5 |
| Polyethylene powder | 1.0 |
| Titanium oxide | 16.0 |
| Polyethylene-treated Red ocher | 0.8 |
| Polyethylene-treated yellow iron oxide | 3.5 |
| Polyethylene-treated black iron oxide | 0.3 |
| Polyethylene-treated kaolin | 10.0 |
| Nylon powder | 3.0 |
| Poly(alkyl acrylate) | 10.0 |

Utilizability in industry

As explained in detail above, it is clear that the oily makeup cosmetics of the present invention are excellent in gloss and feeling at the time of coating and storage stability.

## Claims

**1.** Oily makeup cosmetics comprising an oligo ester obtained from the following components:

(a) behenic acid,
(b) eicosanoic diacid, and
(c) glycerol or a glycerol condensate,

and a polybutene or a Hydrogenated polybutene.

**2.** The oily makeup cosmetics according to Claim 1, wherein a formulation amount of the oligo ester is 0.5 to 10% by weight, and a formulation amount of the polybutene or the hydrogenated polybutene is 5 to 80% by weight.

**3.** The oily makeup cosmetics according to Claim 1, wherein the oligo ester is a material represented by the following formula:

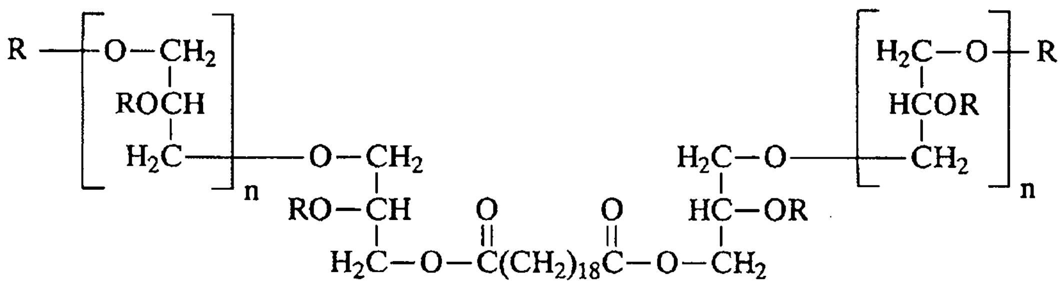

R = 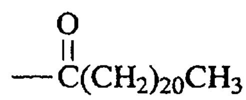 or H

$$n = 0 \sim 8$$

4. The oily makeup cosmetics according to Claim 2, wherein the polybutene or the hydrogenated polybutene is a polybutene or a hydrogenated polybutene having an average molecular weight of 500 to 2700.

5. The oily makeup cosmetics according to Claim 2, wherein a formulation amount of the oligo ester is 1 to 8% by weight based on 100% by weight of the resulting oily makeup cosmetics.

6. The oily makeup cosmetics according to Claim 2, wherein a formulation amount of the oligo ester is 2 to 5% by weight based on 100% by weight of the resulting oily makeup cosmetics.

7. The oily makeup cosmetics according to Claim 2, wherein a formulation amount of the hydrocarbon having a branched structure is 15 to 50% by weight based on 100% by weight of the resulting oily makeup cosmetics.

8. The oily makeup cosmetics according to Claim 2, wherein a formulation amount of the hydrocarbon having a branched structure is 20 to 40% by weight based on 100% by weight of the resulting oily makeup cosmetics.

9. The oily makeup cosmetics according to Claim 4, wherein the polybutene or the hydrogenated polybutene is a polybutene or a hydrogenated polybutene having an average molecular weight of 800 to 1500.

10. The oily makeup cosmetics according to Claim 1, wherein the oily cosmetics do not substantially contain water.

11. The oily makeup cosmetics according to Claim 1, wherein the oily makeup cosmetics further comprises an ultraviolet ray-inhibiting component selected from the group consisting of an inorganic ultraviolet ray-inhibiting component and an organic ultraviolet ray-inhibiting component.

12. The oily makeup cosmetics according to Claim 1, wherein the oily makeup cosmetics comprises 4.0% by weight of (behenic acid/eicosanoic diacid)glyceryl, 25.0% by weight of polybutene, 25.0% by weight of diisostearyl maleate, 5.0% by weight of liquid paraffin, 3.0% by weight of isotridecyl isononanate, 0.4% by weight of Red No. 202, 1.0% by weight of titanium oxide, 5.0% by weight of red ocher-coated titanium-mica and the remainder being 2-ethylhexyl hydroxystearate.

13. The oily makeup cosmetics according to Claim 1, wherein the oily makeup cosmetics comprises 0.5% by weight of glyceryl(behenic acid/eicosanoic diacid), 80.0% by weight of hydrogenated polybutene, 5.0% by weight of $\alpha$-olefin oligomer, 0.1 % by weight of lauroyl lysine-treated Red No. 201, 0.1 % by weight of lauroyl lysine-treated Yellow No. 4, 2.0% by weight of lauroyl lysine-treated titanium-mica and the remainder being 2-ethylhexyl hydroxystearate.

14. The oily makeup cosmetics according to Claim 1, wherein the oily makeup cosmetics comprises 2.0% by weight of

glyceryl(behenic acid/eicosanoic diacid), 40.0% by weight of Hydrogenated polybutene, 20.0% by weight of diisostearyl maleate, 3.0% by weight of methyl phenyl polysiloxanes, 5.0% by weight of α-olefin oligomer, 0.1% by weight of lauroyl lysine-treated Red No. 201, 0.1% by weight of lauroyl lysine-treated Yellow No. 4, 2.0% by weight of lauroyl lysine-treated titanium-mica and the remainder being 2-ethylhexyl hydroxystearate.

**15.** The oily makeup cosmetics according to Claim 1, wherein the oily makeup cosmetics comprises 10.0% by weight of glyceryl(behenic acid/eicosanoic diacid), 2.0% by weight of microcrystalline wax, 2.0% by weight of dextrin palmitate, 5.0% by weight of polybutene, 20.0% by weight of liquid paraffin, 10.0% by weight of jojoba oil, 10.0% by weight of propylene glycol dicaprylate, 5.0% by weight of isononyl isononanate, 10.0% by weight of methylpolysiloxane, 10.0% by weight of silica beads, 3.0% by weight of polyalkyl acrylate, 0.1 % by weight of Red No. 226, 5.0% by weight of (PET/Al/epoxy resin) laminated powder and the remainder being glyceryl trioctanoate.

**16.** The oily makeup cosmetics according to Claim 1, wherein the oily makeup cosmetics comprises 3.0% by weight of titanium-mica, 0.4% by weight of aluminum lake of Red No. 104, 0.5% by weight of aluminum lake of Blue No. 1, 0.2% by weight of Orange No. 201, 8.0% by weight of ceresin, 5.0% by weight of microcrystalline wax, 4.0% by weight of paraffin, 25.0% by weight of hydrogenated polybutene, 2.0% by weight of cetyl isooctanoate, 5.0% by weight of glyceryl(behenate/eicosanoic diacid), 0.5% by weight of vegetable squalane, 0.5% by weight of natural vitamin E, 15.0% by weight of octyl oxystearate, 5.0% by weight of octyldodecanol, 5.0% by weight of tri(caprylic·capric·myristic·stearic acid)glyceride, 10.0% by weight of tri(caprylic·capric acid)glycerin and 10.9% by weight of octyldodecyl ricinoleate.

**17.** The oily makeup cosmetics according to Claim 1, wherein the oily makeup cosmetics comprises 2.0% by weight of candelilla wax, 1.0% by weight of carnauba wax, 0.5% by weight of glyceryl(behenate/eicosanoic diacid), 0.6% by weight of polyethyleneglycol monostearate (45E.O.), 6.0% by weight ofmethylpolysiloxane (20cs), 1.5% by weight of diglycerin oleate, 15.0% by weight of liquid isoparaffin, 5.0% by weight of polybutene, 1.0% by weight of 2-ethylhexyl p-dimethylbenzoatce, 0.5% by weight of anhydrous silicic acid, 1.0% by weight of polyethylene powder, 16.0% by weight of titanium oxide, 0.8% by weight of polyethylene-treated Red ocher, 3.5% by weight of polyethylene-treated yellow iron oxide, 0.3% by weight of polyethylene-treated black iron oxide, 10.0% by weight of polyethylene-treated kaolin, 3.0% by weight of nylon powder, 10.0% by weight of poly(alkyl acrylate) and the remainder being isopropyl isostearate.

## Patentansprüche

**1.** Ölige Make-up-Kosmetik, umfassend einen Oligoester, der aus den folgenden Komponenter erhalten wird:

(a) Behensäure,
(b) Eicosandisäure und
(c) Glycerin oder einem Glycerinkondensat,

und ein Polybuten oder ein hydriertes Polybuten.

**2.** Ölige Make-up-Kosmetik gemäß Anspruch 1, wobei die Formulierungsmenge des Oligoesters 0,5 bis 10 Gew.-% ist und die Formulierungsmenge des Polybutens oder des hydrierten Polybutens 5 bis 80 Gew.-% ist.

**3.** Ölige Make-up-Kosmetik gemäß Anspruch wobei der Oligoester ein Material ist, das durch die folgende Formel dargestellt wird:

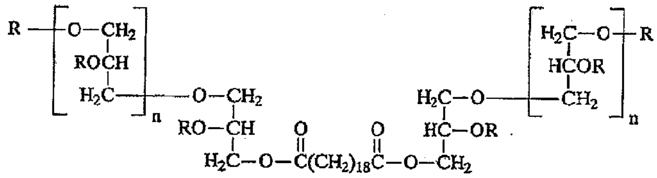

R = 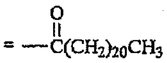

oder H

$n = 0 \sim 8$

4. Ölige Make-up-Kosmetik gemäß Anspruch 2, wobei das Polybuten oder das hydrierte Polybuten ein Polybuten oder ein hydriertes Polybuten mit einem durchschnittlichen Molekulargewicht von 500 bis 2700 ist.

5. Ölige Make-up-Kosmetik gemäß Anspruch 2, wobei die Formulierungsmenge des Oligoesters 1 bis 8 Gew.-%, bezogen auf 100 Gew.-% der resultierenden öligen Make-up-Kosmetik, ist.

6. Ölige Make-up-Kosmetik gemäß Anspruch 2, wobei die Formulierungsmenge des Oligoesters 2 bis 5 Gew.-%, bezogen auf 100 Gew.-% der resultierenden öligen Make-up-Kosmetik, ist.

7. Ölige Make-up-Kosmetik gemäß Anspruch 2, wobei die Formulierungsmenge des Kohlenwasserstoffs mit verzweigter Struktur 15 bis 50 Gew.-% bezogen auf 100 Gew.-% der resultierenden öligen Make-up-Kosmetik, ist.

8. Ölige Make-up-Kosmetik gemäß Anspruch 2, wobei die Formulierungsmenge des Kohlenwasserstoffs mit verzweigter Struktur 20 bis 40 Gew.-%, bezogen auf 100 Gew.-% der resultierenden öligen Make-up-Kosmetik, ist.

9. Ölige Make-up-Kosmetik gemäß Anspruch 4, wobei das Polybuten oder das hydrierte Polybuten ein Polybuten oder ein hydriertes Polybuten mit einem durchschnittlichen Molekulargewicht von 800 bis 1500 ist.

10. Ölige Make-up-Kosmetik gemäß Anspruch 1, wobei die öligen Kosmetik im Wesentlichen kein Wasser enthält.

11. Ölige Make-up-Kcsmetik gemäß Anspruch 1, wobei die ölige Make-up-Kosmetik außerdem eine ultraviolette Strahleninhibierende Komponente umfasst, die aus der Gruppe, bestehend aus einer anorganischen ultraviolette Strahlen-inhibierenden Komponente und einer organischen ultraviolette Strahlen-inhibierenden Komponente, ausgewählt ist.

12. Ölige Make-up-Kosmetik gemäß Anspruch 1, wobei die ölige Make-up-Kosmetik 4,0 Gew.-% (Behensäure/Eicosandisäure)-glyceryl, 25,0 Gew.-% Polybuten, 25,0 Gew.-% Diisostearylmaleat, 5,0 Gew.-% flüssiges Paraffin, 3,0 Gew.-% Isotridecylisononanat, 0,4 Gew.-% Rot Nr. 202, 1,0 Gew.-% Titanoxid, 5,0 Gew.-% Rotocker-beschichteten Titanglimmer und als Rest 2-Ethylhexylhydroxystearin umfasst.

13. Ölige Make-up-Kosmetik gemäß Anspruch 1, wobei die ölige Make-up-Kosmetik 0,5 Gew.-% Glyceryl-(behensäure/eicosandisäure), 80,0 Gew.-% hydriertes Polybuten, 5,0 Gew-% $\alpha$-Olefinoligomer, 0,1 Gew.-% Lauroyllysin-behandeltes Rot Nr. 201, 0,1 Gew.-% Lauroyllysin-behandeltes Gelb Nr. 4, 2,0 Gew.-% Lauroyllysinbehandelten Titanglimmer und als Rest 2-Ethylhexylhydroxystearin umfasst.

14. Ölige Make-up-Kosmetik gemäß Anspruch 1, wobei die ölige Make-up-Kosmetik 2,0 Gew.-% Glyceryl-(behensäure/eicosandisäure), 40,C Gew.-% hydriertes Polybuten, 20,0 Gew.-% Diisostearylmaleat, 3,0 Gew.-% Methylphenylpolysiloxane, 5,0 Gew.-% $\alpha$-Olefinoligomer, 0,1 Gew.-% Lauroyllysin-behandeltes Rot Nr. 201, 0,1 Gew.-% Lauroyllysin-behandeltes Gelb Nr. 4, 2,0 Gew.-% Lauroyllysinbehandelten Titanglimmer und als Rest 2-Ethylhexylhydroxystearat umfasst.

15. Ölige Make-up-Kosmetik gemäß Anspruch 1, wobei die ölige Make-up-Kosmetik 10,0 Gew.-% Glyceryl-(behensäure/eicosandisäure), 2,0 Gew.-% mikrokrisallines Wachs, 2,0 Gew.-% Dextrinpalmitat, 5,0 Gew.-% Polybuten, 20,0 Gew.-% flüssiges Paraffin, 10,0 Gew.-% Jojobaöl, 10,0 Gew.-% Propylenglykoldicaprylat, 5,0 Gew.-% Isononylisononanat, 10,0 Gew.-% Methylpolysiloxan, 10,0 Gew.-% Silikaperlen, 3,0 Gew.-% Polyalkylacrylat, 0,1 Gew.-% Rot Nr. 226, 5,0 Gew.-% (PET/Al/Epoxid-Harz)-laminiertes Pulver und als Rest Glyceryltrioctanoat umfasst.

**16.** Ölige Make-up-Kosmetik gemäß Anspruch 1, wobei die ölige Make-up-Kosmetik 3,0 Gew.-% Titanglimmer, 0,4 Gew.-% Aluminiumlack von Rot Nr. 104, 0,5 Gew.-% Aluminiumlack von Blau Nr. 1, 0,2 Gew.-% Orange Nr. 201, 8,0 Gew.-% Ceresin, 5,0 Gew.-% mikrokristallines Wachs, 4,0 Gew.-% Parafin, 25,0 Gew.-% hydriertes Polybuten, 2,0 Gew.-% Cetylisooctanoat, 5,0 Gew.-% Glyceryl-(behenat/eicosandisäure), 0,5 Gew.-% pflanzliches Squalan, 0,5 Gew.-% natürliches Vitamin E, 15,0 Gew.-% Octyloxystearat, 5,0 Gew.-% Octyldodecanol, 5,0 Gew.-% Tri (capryl-caprin-myristin-stearinsäure)-glycerid, 10,0 Gew.-% Tri(capryl-caprinsäure)glycerin und 10,9 Gew.-% Octyldodecylricinoleat umfasst.

**17.** Ölige Make-up-Kosmetik gemäß Anspruch 1, wobei die ölige Make-up-Kosmetik 2,0 Gew.-% Candelillawachs, 1,0 Gew.-% Carnaubawachs, 0,5 Gew.-% Glyceryl-(behenat/eicosandisäure), 0,6 Gew.-% Polyethylenglykolmonostearat (45E.0.), 6,0 Gew.-% Methylpolysiloxan (20cs), 1,5 Gew.-% Diglycerinoleat, 15,0 Gew.-% flüssiges Isoparaffin, 5,0 Gew.-% Polybuten, 1,0 Gew.-% 2-Ethylhexyl-p-dimethylbenzoat, 0,5 Gew.-% wasserfreie Kieselsäure, 1,0 Gew.-% Polyethylenpulver, 16,0 Gew.-% Titanoxid, 0,8 Gew.-% Polyethylen-behandeltes Rotocker, 3,5 Gew.-% Polyethylen-behandeltes gelbes Eisenoxid, 0,3 Gew.-% Polyethylen-behandeltes schwarzes Eisenoxid, 10,0 Gew.-% Polyethylen-behandelter Kaolin, 3,0 Gew.-% Nylonpulver, 10,0 Gew.-% Poly(alkylacrylat) und als Rest Isopropylisostearat umfasst.

## Revendications

**1.** Produit cosmétique de maquillage à base d'huile comprenant un oligoester obtenu à partir des composants suivants :

(a) l'acide béhénique,
(b) l'acide eicosanoïque, et
(c) le glycérol ou un condensat de glycérol,

et un poly(butène) ou un poly(butène) hydrogéné.

**2.** Produit cosmétique de maquillage à base d'huile selon la revendication 1, dans lequel une quantité de formulation de l'oligoester est de 0,5 à 10 % en poids, et une quantité de formulation du poly(butène) ou du poly(butène) hydrogéné est de 5 à 80 % en poids.

**3.** Produit cosmétique de maquillage à base d'huile selon la revendication 1, dans lequel l'oligoester est une matière représentée par la formule suivante :

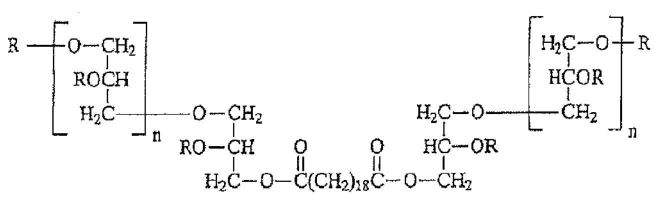

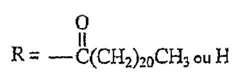

n= 0 ~ 8

**4.** Produit cosmétique de maquillage à base d'huile selon la revendication 2, dans lequel le poly(butène) ou le poly (butène) hydrogéné est un poly(butène) ou un poly(butène) hydrogéné ayant une masse moléculaire moyenne de 500 à 2 700.

**5.** Produit cosmétique de maquillage à base d'huile selon la revendication 2, dans lequel une quantité de formulation de l'oligoester est de 1 à 8 % en poids pour 100 % en poids du produit cosmétique de maquillage à base d'huile résultant.

6. Produit cosmétique de maquillage à base d'huile selon la revendication 2, dans lequel une quantité de formulation de l'oligoester est de 2 à 5 % en poids pour 100 % en poids du produit cosmétique de maquillage à base d'huile résultant.

7. Produit cosmétique de maquillage à base d'huile selon la revendication 2, dans lequel une quantité de formulation de l'hydrocarbure ayant une structure ramifiée est de 15 à 50 % en poids pour 100 % en poids du produit cosmétique de maquillage à base d'huile résultant.

8. Produit cosmétique de maquillage à base d'huile selon la revendication 2, dans lequel une quantité de formulation de l'hydrocarbure ayant une structure ramifiée est de 20 à 40 % en poids pour 100 % en poids du produit cosmétique de maquillage à base d'huile résultant.

9. Produit cosmétique de maquillage à base d'huile selon la revendication 4, dans lequel le poly(butène) ou le poly (butène) hydrogéné est un poly(butène) ou un poly(butène) hydrogéné ayant une masse moléculaire moyenne de 800 à 1 500.

10. Produit cosmétique de maquillage à base d'huile selon la revendication 1, dans lequel le produit cosmétique à base d'huile ne contient sensiblement pas d'eau.

11. Produit cosmétique de maquillage à base d'huile selon la revendication 1, dans lequel le produit cosmétique de maquillage à base d'huile comprend en outre un composant inhibant les rayons ultraviolets choisi dans le groupe constitué par un composant inhibant les rayons ultraviolets inorganique et un composant inhibant les rayons ultraviolets organique.

12. Produit cosmétique de maquillage à base d'huile selon la revendication 1, dans lequel le produit cosmétique de maquillage à base d'huile comprend 4,0 % en poids de (acide béhénique/diacide eicosanoïque)glycéryle, 25,0 % en poids de poly(butène), 25,0 % en poids de maléate de diisostéaryle, 5,0 % en poids de paraffine liquide, 3,0 % on poids d'isononanate d'isotridécyle, 0,4 % en poids de Rouge n° 202, 1,0 % en poids d'oxyde de titane, 5,0 % en poids de titane-mica revêtu de rouge ocre et le reste étant constitué de l'hydroxystéarate de 2-éthylhexyle.

13. Produit cosmétique de maquillage à base d'huile selon la revendication 1, dans lequel le produit cosmétique de maquillage à base d'huile comprend 0,5 % en poids de glycéryl(acide béhénique/diacide eicosanoïque), 80,0 % en poids de poly(butène) hydrogéné, 5,0 % en poids d'un oligomère d'$\alpha$-oléfine, 0,1 % en poids de Rouge n° 201 traité à la lauroyl lysine, 0,1 % en poids de Jaune n° 4 traité à la lauroyl lysine, 2,0 % en poids d'oxyde de titane-mica traité à la lauroyl lysine et le reste étant constitué de l'hydroxystéarate de 2-éthylhexyle.

14. Produit cosmétique de maquillage à base d'huile selon la revendication 1, dans lequel le produit cosmétique de maquillage à base d'huile comprend 2,0 % en poids de glycéryl(acide béhérique/diacide eicosanoïque), 40,0 % en poids de poly(butène) hydrogéné, 20,0 % en poids de maléate de diisostéaryle, 3,0 % en poids de méthylphénylpolysiloxanes, 5,0 % en poids d'un oligomère d'$\alpha$-oléfine, 0,1 % en poids de Rouge n° 201 traité à la lauroyl lysine, 0,1 % en poids de Jaune n° 4 traité à la lauroyl lysine, 2,0 % en poids d'oxyde de titane-mica traité à la lauroyl lysine et le reste étant constitué de l'hydroxystéarate de 2-éthylhexyle.

15. Produit cosmétique de maquillage à base d'huile selon la revendication 1, dans lequel le produit cosmétique de maquillage à base d'huile comprend 10,0 % en poids de glycéryl(acide béhénique/diacide eicosanoïque), 2,0 % en poids de cire microcristalline, 2,0 % en poids de palmitate de dextrine, 5,0 % en poids de poly(buténe), 20,0 % en poids de paraffine liquide, 10,0 % en poids d'huile de jojoba, 10,0 % en poids de dicaprilate de propylène glycol, 5,0 % en poids d'isononanate d'isononyle, 10,0 % en poids de méthylpolysiloxane, 10,0 % en poids de billes de silice, 3,0 % en poids de poly(acrylate d'alkyle), 0,1 % en poids de Rouge n° 226, 5,0 % en poids de poudre stratifiée (PET/Al/résine époxy) et le reste étant constitué de trioctanoate de glycéryle.

16. Produit cosmétique de maquillage à base d'huile selon la revendication 1, dans lequel le produit cosmétique de maquillage à base d'huile comprend 3,0 % en poids de titane-mica, 0,4 % en poids de laque d'aluminium de Rouge n° 104, 0,5 % en poids de laque d'aluminium de Bleu n° 1, 0,2 % en poids d'Orange n° 201, 8,0 % en poids de cérésine, 5,0 % en poids de cire microcristalline, 4,0 % en poids de paraffine, 25,0 % en poids de poly(butène) hydrogéné, 2,0 % en poids d'isooctanoate de cétyle, 5,0 % en poids de glycéryl(béhénate/diacide eicosanoïque), 0,5 % en poids de squalane végétal, 0,5 % en poids de vitamine E naturelle, 15,C % en poids d'oxystéarate d'octyle, 5,0 % en poids d'octyldodécanol, 5,0 % en poids de tri(acide caprylique·caprique·myristique.stéarique) glycéride,

10,0 % en poids de tri(acide caprylique·caprique) glycérine et 10,9 % en poids de ricinoléate d'octyldodécyle.

**17.** Produit cosmétique de maquillage à base d'huile selon la revendication 1, dans lequel le produit cosmétique de maquillage à base d'huile comprend 2,0 % en poids de cire de candelilla, 1,0 % en poids de cire de carnauba, 0,5 % en poids de glycéryl(béhénate/diacide eicosanoïque), 0,6 % en poids de monostérate de poly(éthylène glycol) (45E.O), 6,0 % en poids de méthylpolysiloxane (20cs), 1,5 % en poids d'oléate de diglycérine, 15,0 % en poids d'isoparaffine liquide, 5,0 % en poids de poly(butène), 1,0 % en poids de p-diméthylbenzoate de 2-éthylhexyle, 0,5 % en poids d'acide silicique anhydre, 1,0 % en poids de poudre de poly(éthylène), 16,0 % en poids d'oxyde de titane, 0,8 % en poids de Rouge ocre traité au poly(étrylène), 3,5 % en poids d'oxyde de fer jaune traité au poly (éthylène), 0,3 % en poids d'oxyde de fer noir traité au poly(éthylène), 10,0 % en poids de kaolin traité au poly (éthylène), 3,0 % en poids de poudre de nylon, 10,0 % en poids de poly(acrylate d'alkyle), le reste étant constitué d'isostéarate d'isopropyle.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP SHOU617402 B
- JP HEI7126604 B
- JP 2000219617 A